# EUROPEAN PATENT SPECIFICATION

(11) **EP 0 300 674 B1**
(45) Date of publication and mention of the grant of the patent: **31.03.1993**
(21) Application number: 88306390.1
(22) Date of filing: 13.07.1988
(51) Int. Cl.: C07D 493/22, A61K 31/365, A23K 1/17, A01N 43/90, C12P 17/08

(54) **Directed biosynthesis of milbemycin analog**
Gerichtete-Biosynthese eines Milbemycinanaloges
Bio-synthèse dirigée d'analogues de la milbémycine

(30) Priority: 20.07.1987 US 75474
(43) Date of publication of application: 25.01.1989
(73) Proprietor: Merck & Co., Inc., Rahway New Jersey 07065-0900 (US)
(72) Inventor: Chen, Shieh-Shung T., Morganville New Jersey 07751 (US)
(74) Representative: Cole, William Gwyn

(56) References cited:
- EP-A- 0 170 006
- EP-A- 0 214 731

## Description

### BACKGROUND OF THE INVENTION

In EPO application 85106844 (publication 170006) are disclosed derivatives of milbemycin compounds which are unsaturated in a 25-position side chain. The unsaturated side chain is from 4 to 6 carbon atoms in length with the longest chain being a 1,3-dimethyl-1-butene group. The compounds are prepared from the fermentation of a microorganism identified as Streptomyces cyanogriseus subsp. noncyanogenus NRRL-15773. Several of the same compounds described in EPO 85106844 are also described in U.K. patent application 2166436A which are prepared from the fermentation of the microorganism Streptomyces thermoarchaensis NC1B 12015. Such compounds also have 25-position unsaturated side chains of from 4 to 6 carbon atoms.

EP-A-0214731 describes a fermentation process for preparing antiparasitic avermectin and milbemycin derivatives having a non-natural substituent group R² at the 25-position, which comprises adding to the fermentation medium a carboxylic acid derivative R²-CO₂H, or a salt, ester or amide thereof or oxidative precursor therefor. Also disclosed in this publication is a range of non-natural avermectin and milbemycin derivatives obtained from the described fermentation process.

### DISLOSURE OF THE INVENTION

An example of the compounds disclosed in EPO 85106844 is seen in the following structure: 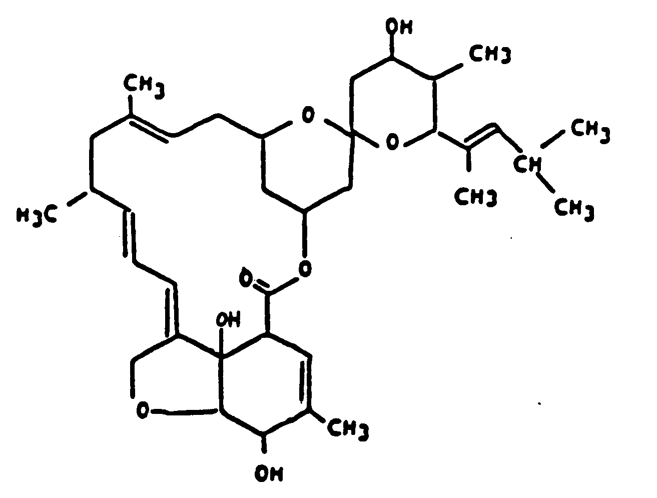
which is identified as LL-F28249α. This compound and others are isolated from the fermentation broth of a culture of Streptomyces cyanogriseus subsp. noncyanogenus. This culture is fully described in the above-identified application and a sample is publicly available under the accession number NRRL 15773.

The instant compound is an analog of the above compound and is described in the following structure:

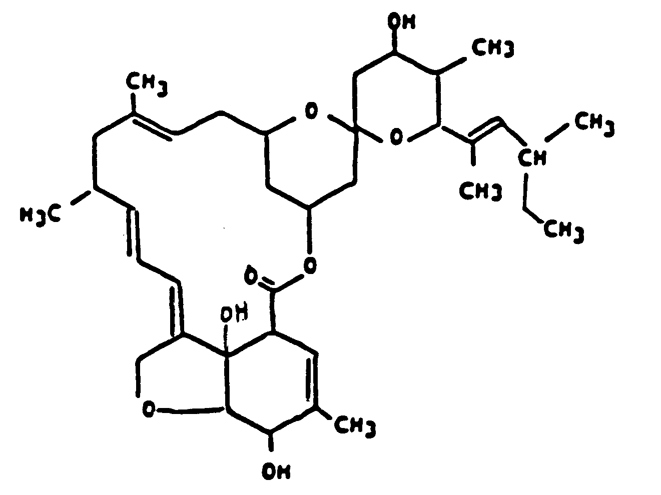

The above compound is prepared using the same (NRRL 15773) culture used to prepare LL-F 28249α, however, the normal biosynthetic pathway is forced into a modified pathway by adding to the fermentation culture a quantity of 2-methylbutyric acid. The added 2-methylbutyric acid incorporates itself into the molecules being prepared by the culture and produces the analogous compound with an unsaturated seven membered chain at the 25-position rather than the six membered chain.

The process may be carried out using assimilable sources of carbon, nitrogen and inorganic salts such as the fermentation media described in EPO 85106844, or in other culture media suitable for Streptomyces fermentation. The 2-methylbutyric acid, which may also be used as 2-methylbutyrate salts which are non-toxic to the culture, such as alkali metal salts, preferably the sodium salt, is added to the culture medium at any time during the course of the fermentation. However, in order to allow the 2-methylbutyric acid to be adequately taken into the biosynthetic pathway, it is preferred to add it during the first half of the fermentation period. Generally the fermentation is complete in from 150 to 300 hours, thus the 2-methylbutyric acid addition would be completed before the 25 to 150 hour point. It has been further found advantageous to add the 2-methylbutyric acid to the culture medium in from 2 to 6 divided portions during the first half of the fermentation period.

The 2-methylbutyric acid is added to the fermentation medium at a level of from 0.1 to 2.0 g per liter of the medium, preferably from 0.3 to 0.8 g/l and most preferably at about 0.5 g/l.

The product is isolated from the fermentation broth using techniques known for the isolation of macrocyclic compounds from Streptomyces broths such as those described in EPO 85106844 or in U.K. 2166436A.

The novel compound of this invention has parasiticidal activity as an anthelmintic, ectoparasiticide, insecticide and acaricide, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Parascaris. Certain of these, such as Nematodirus, Cooperia and Oesophagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The compound of this invention has activity against these parasites, and in addition is also active against Dirofilaria in dogs, Nematospiroides, Syphacia, Aspiculuris in rodents, arthropod ectoparasites of animals and birds such as ticks, mites, lice, fleas, blowfly, in sheep Lucilia sp., biting insects and such migrating dipterous larvae as Hypoderma sp. cattle, Gastrophilus in horses, and Cuterebra sp. in rodents.

The instant compound is also useful against parasites which infect humans. The most common genera of parasties of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra intestinal stages of the intestinal worms Strongyloides and Trichinella. The compound is also of value against arthropods parasitizing man, biting insects and other dipterous pests causing annoyance to man.

The compound is also active against household pests such as the cockroach, Blatella sp., clothes moth, Tineola sp., carpet beetle, Attagenus sp., and the housefly Musca domestica.

The compound is also useful against insect pests of stored grains such as Tribolium sp., Tenebrio sp. and of agricultural plants such as two-spotted spider mites, (Tetranychus sp.), aphids, (Acyrthiosiphon sp.); against migratory orthopterans such as locusts and immature stages of insects living on plant tissue. The compound is useful as a nematocide for the control of soil nematodes and plant parasites such as Meloidogyne spp. which may be of importance in agriculture The compound is active against other plant pests such as the southern army worm and Mexican bean beetle larvae.

The compound may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contains from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the compound in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be adminis tered via an animal feedstuff, it is intimately dispersed in the feed or used as a top dressing or in the form of pellets which may then be added to the finished feed or optionally fed separately. Alternatively, the antiparasitic compound may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol formal, and aqueous parenteral formulations are also used. The active compound is dissolved or suspended in the parenteral formulation for administration; such formulations generally contain from 0.005 to 5% by weight of the active compound.

Although the antiparasitic agent of this invention finds its primary use in the treatment and/or prevention of helminthiasis, they are also useful in the prevention and treatment of diseases caused by other parasites, for example, arthropod parasites such as ticks, lice, fleas, mites and other biting insects in domesticated animals and poultry. It is also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compound by administering about 0.001 to 10 mg of drug per kg of animal body weight, such total dose being given at one time or in divided doses over a relatively short period of time such as 1-5 days. With the compound of this invention, control of such parasties is obtained in animals by administering from about 0.025 to 1 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compound described herein is administered as a component of the feed of the animals, or dissolved or suspended in the drinking water, compositions are provided in which the active compound is intimately dispersed in an inert carrier or diluent. An inert carrier is one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active hydrogenated avermectin compound is intimately dispersed throughout the carrier by methods such as grinding, stirring, milling or tumbling. Compositions containing from about 0.005 to 2.0% by weight of the active compound are particularly suitable as feed premixes. Feed supplements, which are fed directly to the animal, contain from about 0.0002 to 0.3% by weight of the active compound.

Such supplements are added to the animal feed in an amount to give the finished feed the concentration of active compound desired for the treatment and control of parastic diseases. Although the desired concentration of active compound will vary depending upon the factors previously mentioned, the compound of this invention is usually fed at concentrations of between 0.00001 to 0.002% in the feed in order to achieve the desired antiparasitic result.

The compound of this invention is also useful in combatting agricultural pests that inflict damage upon crops while they are growing or while in storage. The compound is applied using known techniques as sprays, dusts, emulsions and the like, to the growing or stored crops to effect protection from such agricultural pests.

The following examples are provided in order that the invention may be more fully understood. They are not to be construed as being limitative of the invention.

### EXAMPLE 1

### Fermentation

A seed medium is prepared using the following ingredients:

| Seed Medium | g/l |
|---|---|
| Glucose | 20 |
| Hycase | 20 |
| Yeast extract | 20 |
| KNO₃ | 2.0 |
| FeSO₄·7H₂O | 0.025 |
| NaCl | 0.5 |
| MgSO₄ | 0.5 |
| MnSO₄ | 0.005 |
| ZnSO₄ | 0.01 |
| CaCl₂·2H₂O | 0.02 |
| pH 7.0 | |

A frozen vial of Streptomyces cyanogriseus subsp. noncyanogenus (NRRL 15773) is sterile transferred to a 250 ml Erlenmeyer flask containing 25 ml of seed medium and incubated on a rotary shaker at 27°C for 24 hours.

Fermentation medium is prepared using the following ingredients:

| Fermentation Medium | g/l |
|---|---|
| Glucose | 40 |
| Peptonized milk | 1 |
| Hy Soy Type T | 5 |
| CaCO₃ | 1 |
| pH 7.2 | |

A one ml sample of the seed medium is transferred to each of 10 fermentation flasks (250 ml) containing 20 ml of fermentation medium. The culture is incubated at 27°C on a rotary shaker for 192 hours (8 days). A filter sterilized solution of 2-methylbutyrate sodium salt is added to the fermentation flasks in portions after 48, 72 and 96 hours of fermentation such that the final concentration of 2-methylbutyric acid is 0.5 g/l.

### EXAMPLE 2

### Isolation and Purification

The mycelial cake from 200 ml whole broth from the fermentation of Example 1 is collected by centrifugation and washed with 200 ml of water. The collected wet cake is extracted three times with 50 ml portions of methanol. The methanol extracts are pooled and taken to dryness under vacuum. The residue is dissolved in methylene chloride and adsorbed on a silica gel column (Mallinchrodt Silic AR 100-20 mesh). The column is developed with methylene chloride and the product is eluted with 10% isopropyl alcohol in methylene chloride. The eluates are evaporated in vacuo to give a dark yellowish residue (200 mg). The sample is then separated by preparative high performance liquid chromatography (HPLC) on an E. Dupont Zorbar C-18 reverse phase 21.2 mm x 25 cm column developing at 6 ml/min. with a 83/17 methanol/water system to yield 2.5 mg of pure product.

## Claims

1. A process for the preparation of a compound having the formula 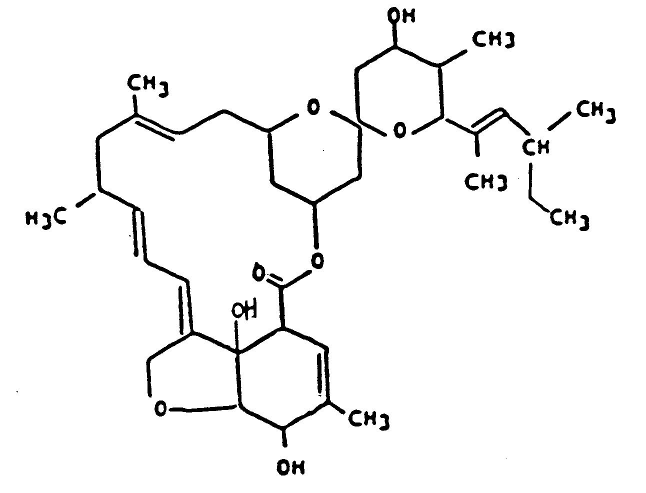 which comprises fermenting in a medium of assimilable sources of carbon, nitrogen and inorganic salts, a strain of Streptomyces cyanogriseus subsp. noncyanogenus, NRRL 15773 capable of producing said compound, and adding to the fermentation medium 2-methylbutyric acid or salts thereof.

2. The process of Claim 1 wherein the 2-methylbutyric acid is added during the first half of the fermentation period.

3. The process of Claim 1 wherein the 2-methylbutyric acid or salt thereof is added at a concentration of 0.1 to 2.0 g per liter of fermentation medium.

4. The process of Claim 3 wherein the 2-methylbutyric acid or salt thereof is added at a concentration of 0.3 to 0.8 g per liter of fermentation medium.

5. The process of Claim 4 wherein the 2-methylbutyric acid is added at a concentration of about 0.5 g per liter of fermentation medium.

6. The process of Claim 1 wherein the 2-methylbutyric acid is added in from 2 to 6 divided portions.

7. The process of Claim 1 wherein the 2-methylbutyric acid is added as the alkali metal salt.

8. The process of Claim 7 wherein the 2-methylbutyric acid is added as the sodium salt.

## Patentansprüche

1. Verfahren zur Herstellung einer Verbindung der Formel 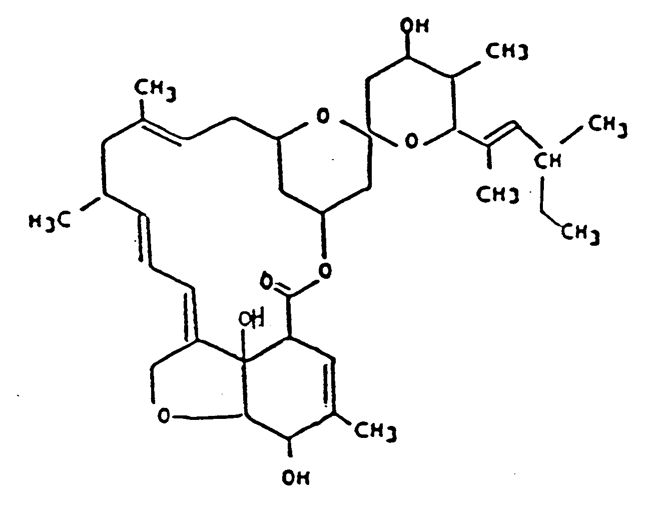 das die Fermentation eines Stammes von Streptomyces cyanogriseus subsp. noncyanogenus, NRRL 15773, der in der Lage ist, die genannte Verbindung zu produzieren, in einem Medium von assimilierbaren Kohlenstoff-, Stickstoff- und anorganischen Salzquellen und die Zugabe von 2-Methylbuttersäure oder Salzen davon zu dem Fermentationsmedium umfaßt.

2. Verfahren nach Anspruch 1, bei dem die 2-Methylbuttersäure während der ersten Hälfte des Fermentationszeitraumes zugegeben wird.

3. Verfahren nach Anspruch 1, bei dem die 2-Methylbuttersäure oder die Salze davon in einer Konzentration von 0,1 bis 2,0 g pro Liter Fermentationsmedium zugegeben werden.

4. Verfahren nach Anspruch 3, bei dem die 2-Methylbuttersäure oder die Salze davon in einer Konzentration von 0,3 bis 0,8 g pro Liter Fermentationsmedium zugegeben werden.

5. Verfahren nach Anspruch 4, bei dem die 2-Methylbuttersäure in einer Konzentration von etwa 0,5 g pro Liter Fermentationsmedium zugegeben wird.

6. Verfahren nach Anspruch 1, bei dem die 2-Methylbuttersäure in 2 bis 6 aufgeteilten Portionen zugegeben wird.

7. Verfahren nach Anspruch 1, bei dem die 2-Methylbuttersäure als Alkalimetallsalz zugegeben wird.

8. Verfahren nach Anspruch 7, bei dem die 2-Methylbuttersäure als Natriumsalz zugegeben wird.

## Revendications

1. Procédé de préparation d'un composé de formule 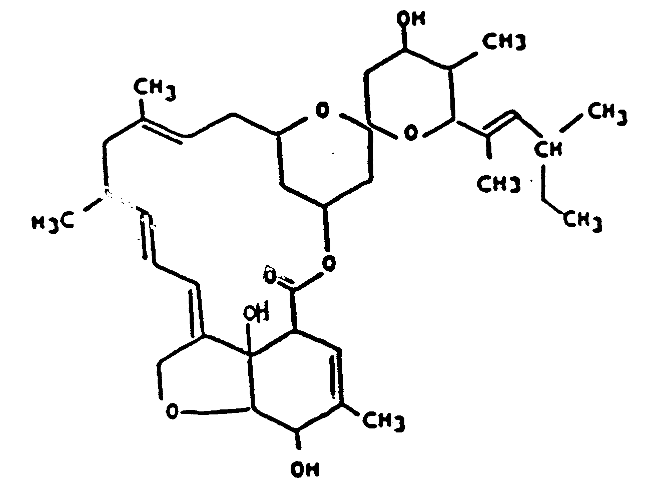 qui comprend de faire fermenter dans un milieu de sources assimilables, de carbone, d'azote et de sels inorganiques, une souche de Streptomyces cyanogriseus noncyanogenus, NRRL-15773 capable de produire de tels composés et d'ajouter au milieu de fermentation de l'acide 2-méthylbutyrique ou des sels de celui-ci.

2. Le procédé de la Revendication 1 dans lequel on ajoute l'acide 2-méthylbutyrique au cours de la première moitié de la période de fermentation.

3. Le procédé de la Revendication 1 dans lequel on ajoute l'acide 2-méthylbutyrique ou un sel de celui-ci à une concentration de 0,1 à 2,0 g par litre de milieu de fermentation.

4. Le procédé de la Revendication 3 dans lequel on ajoute l'acide 2-méthylbutyrique ou un sel de celui-ci à une concentration de 0,3 à 0,8 g par litre de milieu de fermentation.

5. Le procédé de la Revendication 4 dans lequel on ajoute l'acide 2-méthylbutyrique à une concentration d'environ 0,5 g par litre de milieu de fermentation.

6. Le procédé de la Revendication 1 dans lequel on ajoute l'acide 2-méthylbutyrique divisé en deux à six portions.

7. Le procédé de la Revendication 1 dans lequel on ajoute l'acide 2-méthylbutyrique sous la forme de son sel de métal alcalin.

8. Le procédé de la Revendication 1 dans lequel on ajoute l'acide 2-méthylbutyrique sous la forme de son sel de sodium.
